Europäisches Patentamt

(19) European Patent Office

Office européen des brevets

(11) **EP 0 526 608 B1**

(12) **FASCICULE DE BREVET EUROPEEN**

(45) Date de publication et mention
de la délivrance du brevet:
**18.12.1996 Bulletin 1996/51**

(21) Numéro de dépôt: **92905338.7**

(22) Date de dépôt: **24.02.1992**

(51) Int. Cl.$^6$: **A61K 31/49**

(86) Numéro de dépôt international:
**PCT/EP92/00408**

(87) Numéro de publication internationale:
**WO 92/14467 (03.09.1992 Gazette 1992/23)**

(54) **AGENTS THERAPEUTIQUES POUR LE TRAITEMENT DE LA RESISTANCE MULTIDROGUE DE CANCERS**

Therapeutische Mittel für die Behandlungder Resistenz gegen Arzneimittel bei Krebs

THERAPEUTIC AGENTS FOR THE TREATMENT OF MULTIDRUG RESISTANCE TO CANCERS

(84) Etats contractants désignés:
**AT BE CH DE DK ES FR GB GR IT LI LU MC NL SE**

(30) Priorité: **25.02.1991 CH 576/91**
**02.12.1991 CH 3522/91**

(43) Date de publication de la demande:
**10.02.1993 Bulletin 1993/06**

(73) Titulaires:
• **DEBIOPHARM S.A.**
**CH-1003 Lausanne (CH)**
• **PATRINOVE**
**F-69006 Lyon (FR)**
• **INSTITUT NATIONAL DE LA SANTE ET DE LA RECHERCHE MEDICALE (INSERM)**
**75654 Paris Cédex 13 (FR)**

(72) Inventeurs:
• **CHAUFFERT, Bruno**
**F-21000 Dijon (FR)**
• **GENNE, Philippe**
**F-21121 Ahuy (FR)**
• **GUTIERREZ, Gilles**
**F-69006 Lyon (FR)**
• **MAUVERNAY, Rolland-Yves**
**CH-1003 Lausanne (CH)**

(74) Mandataire: **Micheli & Cie**
**Rue de Genève 122,**
**Case Postale 61**
**1226 Genève-Thonex (CH)**

(56) Documents cités:
• **H. RIMPLER et al.: "Pharmazeutische Biologie II. Biogene Arzneistoffe", 1990, pages 437-440: "Cinchonae cortex", Georg Thieme Verlag, Stuttgart, DE, voir pages 439-440**
• **J.E.F. REYNOLDS et al.: "Martindale - The Extra Pharmacopoeia", 28 édition, 1982, page 1378, The Pharmaceutical Press, Londres, GB, voir monographie no. 7786-v: "Hydroquinidine hydrochloride"**
• **S. BUDAVARI et al.: "The Merck Index", 11 édition, 1989, page 356, Merck & Co., Rahway, NJ, US, voir monographie no 2289: "Cinchonine"**
• **S. BUDAVARI et al.: "The Merck Index", 11 édition, 1989, page 762, Merck & Co., Rahway, NJ, US, voir monographie no. 4736: "Hydroquinidine"**
• **Ann. Ist. Super. Sanita, vol. 21, no. 3, 1985, (IT); D.C. WARHURST: "New drugs and their potential use against drug-resistant malaria", pages 327-336, voir abrégé; pages 328-329**
• **Nature, vol. 345, 17 mai 1990, Londres, GB: C. NEWBOLD: "The path of drug resistance", pages 202-203, voir page 202**
• **Annals of Tropical Medicine and parasitology, vol. 69, no. 4, décembre 1975, Liverpool, GB; N.M. MATTOCK et al.: "The experimental chemotherapy of leishmaniasis. III. Detection of antileishmanial activity in some new synthetic compounds in a tissue culture model", pages 449-462, voir page 456**

- Therapie, vol. 43, no. 4, juin/juillet 1988; B. FLOUVAT et al.: "Métabolites de la dihydroquinidine observés après administration à l'homme d'une formulation à libération prolongée", pages 255-261, voir l'abrégé
- J. Chem. Soc. Perkin Trans. I, 1988; M. IHARA et al.: "Total synthesis of hydrocinchonidine and hydrocinchonine via photo-oxygenation of an indole derivative", pages 1277-1281, voir l'abrégé; schémas 1-4
- Molecular Pharmacology, vol. 33, n 4, avril 1988 (US); J.M.ZAMORA et al.:" Physical-chemical properties shared by compounds that modulate multidrug resistance in human leukemic cells" , pages 454-462, voir abrégé; pages 457-458
- Proceedings of the 77th Annual Meeting of the American Association for Cancer Research, Los Angeles, CA, 7-10 mai 1986, vol. 27; J.M. ZAMORA et al.: "Potentiation of Vinca alkaloid (VA) cytotoxicity by quinolines, acridines, indole alkaloids and aromatic amines", page 395, abrègè no. 1567
- International Journal of Cancer, vol. 46, n 1, 15 juillet 1990, Lausanne, CH; J.F. ELIASON et al.:" Human multi-drug-resistant cancer cells exhibit a high degree of selectivity for stereoisomers of verapamil and quinidine", pages 113- 117, voir le document en entier
- Tetradron Letters, vol. 27, n 49, 1986, (GB); R.T. BROWN et al.:" Stereospecific synthesis of erythro Cinchona alkaloids from secologanin", pages 6005-6008, voir pages 6005-6006
- Cancer Research 52, 1992, pages 2797 - 2801

Remarques:
    Le dossier contient des informations techniques présentées postérieurement au dépôt de la demande et ne figurant pas dans le présent fascicule.

**Description**

L'invention a pour objet l'utilisation de cinchonine dans la préparation de compositions pharmaceutiques destinées au traitement de tumeurs cancéreuses, plus précisement un agent destiné à renforcer l'efficacité de substances cytotoxiques utilisées dans le traitement de tumeurs cancéreuses humaines ou animales développant le phénomène de résistance multiple aux agents anti-cancéreux (résistance multidrogue - "multiple drug resistance : MDR").

Le phénomène de la résistance multidrogue est connu et ne se limite pas seulement aux agents anti-cancéreux. A ce jour, diverses explications sont avancées quant à la compréhension des mécanismes mis en jeu. Pour ce qui est du comportement des cellules tumorales cancéreuses, on a pu mettre en évidence plusieurs systèmes de résistance distincts : s'agissant par exemple de la perméabilité de la membrane cellulaire, l'intervention d'une glyco-protéine spécifique (P-gp) est reconnue, mais il est admis que d'autres facteurs protéiques peuvent intervenir.

L'approche de ce phénomène est donc multiple, sinon complexe, ce qui rend d'autant plus difficile la recherche de solutions appropriées.

L'application de substances ou drogues cytotoxiques dans le traitement des tumeurs cancéreuses se heurte à plusieurs obstacles. La plupart des drogues utilisées à cet effet présentent premièrement une toxicité intrinsèque, non distinctive, source d'effets secondaires néfastes; d'autre part, au vu de cette toxicité intrinsèque, leur administration au patient s'en trouve quantitativement limitée et, dans de nombreux cas, l'activité souhaitée au niveau des tumeurs n'est plus suffisante.

Divers moyens ont été déjà proposés en vue de surmonter de tels obstacles, comme la vectorisation de la substance cytotoxique, par exemple sous forme de son incorporation dans un liposome. Dans un tel cas cependant, si la toxicité intrinsèque de la substance cytotoxique est temporairement occultée et donc avec des effets secondaires limités pour le patient, son activité sur le site tumoral n'est pas forcément rétablie au niveau d'efficacité souhaité.

En outre, pour peu que les cellules tumorales soumises à ce traitement développent le phénomène de résistance multidrogue, innée ou acquise, la substance cytotoxique perd quasiment toute efficacité.

On connaît à ce jour plusieurs substances montrant in vitro une activité inhibitrice de la MDR : ce sont pour l'essentiel des substances non cytotoxiques, généralement de caractère hydrophobe, tels des alcaloïdes par exemple. In vitro, leur utilisation conjointe à une drogue cytotoxique apparaît comme satisfaisante, le phénomène dit MDR étant significativement inhibé au niveau cellulaire pour que la dite drogue joue pleinement son rôle.

Il en est tout autrement, cependant, lorsque l'on tente une transposition de telles observations sur un organisme vivant. Les substances inhibitrices de la MDR, alcaloïdes ou autres, présentent également une toxicité intrinsèque qui limite leur administration en-deçà d'un seuil (taux sérique) où l'activité recherchée (inhibition de la MDR) est également perdue. En outre, bien que la substance cytotoxique soit administrée sous forme libre, ou sous forme de liposome par exemple, on a observé comme facteur limitatif important une augmentation concommittante de la toxicité intrinsèque de la drogue cytotoxique due à un changement significatif de sa distribution pharmacologique dans l'organisme. De fait, face à de tels inconvénients, l'homme du métier se trouve particulièrement démuni, s'agissant du traitement in vivo de tumeurs cancéreuses développant le phénomène de résistance multidrogue (MDR). La quinine, récemment proposée à titre de substance inhibitrice de la MDR, illustre exactement ce cas de figure et son utilisation à des fins thérapeutiques humaines s'en trouve limitée d'autant.

L'invention a le mérite d'apporter une solution originale et particulièrement efficace au problème évoqué plus haut, ceci d'autant plus qu'elle vise l'utilisation de substances de structure voisine de celle de la quinine. De manière surprenante cependant, de telles substances se sont révélées, à dose comparable, plus efficaces et en même temps nettement moins toxiques que la quinine dans le contexte présent. Comme on l'a vu ci-dessus, cette voie semblait cependant compromise, sinon exclue, quant à l'utilisation de tels alcaloïdes à titre de substances inhibitrices de la résistance multiple aux agents anti-cancéreux, sur le plan thérapeutique.

L'invention donc a pour objet l'utilisation de cinchonine à titre de substance inhibitrice de la résistance multidrogue (MDR) dans la préparation de compositions pharmaceutiques destinées au traitement de tumeurs cancéreuses développant le phénomène de résistance multidrogue.

La cinchonine et la dihydrocinchonine sont des substances connues, de la classe des alcaloïdes, proposées comme agents thérapeutiques anti-malaria. L'hydroquinidine, pour sa part, a été proposée comme agent antiarythmisant. La pharmacologie de telles substances est connue; il en va de même pour leur toxicité.

Selon l'invention, la cinchonine peut être avantageusement utilisée sous forme de composition pharmaceutique, solubilisée dans un milieu approprié à une administration parentérale, par exemple la voie sous-cutanée ou intra-musculaire, ou encore sous forme de suspension ou comprimé destiné à l'administration par voie orale. Parmi de telles compositions, la cinchonine peut être également associée à un véhicule particulaire telle une microcapsule ou microparticule, une nanocapsule ou nanoparticule. A titre de véhicule particulier, on peut avantageusement utiliser un liposome.

Au sein des compositions pharmaceutiques selon l'invention, on peut utiliser à titre d'agent inhibiteur de la MDR la cinchonine également en mélange avec, par exemple, la dihydrocinchonine, dans un rapport pondéral variable.

Il est en outre entendu, que de telles substances peuvent se présenter sous forme de stéréoisomères purs ou de mélanges de tels stéréoisomères, respectivement d'énantiomères purs ou de mélange d'énantiomères. S'agissant par exemple de cinchonine ou de dihydrocinchonine, on peut en effet distinguer divers stéréoisomères, selon la configuration en C8 ou en C9.

Selon l'invention également, la cinchonine peut être utilisée conjointement à d'autres substances inhibitrices de la MDR, plus particulièrement des substances dont l'activité inhibitrice a déjà été confirmée. A ce titre, on peut citer de manière non exhaustive, une ou plusieurs substances connues choisies parmi l'amiodarone, la quinine, la quinidine, la cinchonidine, le vérapamil, la cyclosporine A, les céphalosporines, le bipéridène, la lidocaïne, la chlorpromazine, la pentazocine, la prométhazine, le potassium canmrénoate, l'amitriptyline, le propanolol, le déméthoxyvérapamil, le diltiazème, la thioridazine, la trifluopérazine, la chloroquine, la sdb-éthylène diamine, la réserpine, le tamoxifène, le torémifène, l'hydrocortisone, la progestérone, le salbutamol et leurs dérivés acylés ou esters.

Selon l'invention, l'effet inhibiteur souhaité peut être obtenu à des doses auxquelles on n'observe pas chez les sujets traités les effets secondaires néfastes dus à la toxicité intrinsèque de la cinchonine. Bien entendu, les doses thérapeutiques optimales dépendront du type de tumeur traité, de la nature de l'agent cytotoxique utilisé conjointement, du sujet traité ou encore d'autres facteurs que l'homme du métier sera en mesure d'appréhender.

Des substances cytotoxiques sensibles au phénomène de la MDR, dont l'effet peut être avantageusement augmenté par l'action concomitante de la cinchonine, sont mentionnées ci-après; cette énumération n'est pas exhaustive pour autant.

Ce sont pour l'essentiel des substances hydrophobes, ayant en commun un résidu azoté chargé positivement, tels les alcaloïdes de la vinca, les anthracyclines ou produits analogues, les épipodophyllotoxines ou les antibiotiques antitumoraux par exemple. On peut citer en particulier la vincristine, la vinblastine, la vindésine, la vinorelbine, la doxorubicine, la déoxydoxorubicine, la tétrahydropyranyladriamycine, l'épidoxorubicine, l'aclacinomycine, la déméthoxydaunorubicine, la daunorubicine, le m-amsa, la mitoxantrone,le bisanthrène, le déméthoxydaunorubisanthrène, la mithramycine, l'actinomycine D, la puromycine, l'étoposide, le ténoposide, l'émétine, l'éthidium bromide, la cytochalasine, la colchicine et le taxol.

Grâce à l'invention, on est en mesure de proposer un traitement thérapeutique approprié concernant de nombreuses tumeurs cancéreuses présentant à des degrés divers une résistance multiple aux agents anti-cancéreux (MDR). A ce propos, on peut citer entre autres la leucémie aigue myeloblastique, la leucémie aigue lymphoblastique, le neuroblastome, le cancer du poumon à petites cellules, le cancer de l'ovaire, le lymphome malin non hodgkinien et le plasmocytome diffus. Ce sont des cancers qui peuvent présenter une MDR induite, en réponse au traitement avec un agent cytotoxique.

On peut également traiter des cancers présentant une MDR innée ou, pour le moins, des cellules cancéreuses caractérisées par la présence, avant tout traitement, du gène correspondant de la P-gp (mdr 1) à un taux relativement élevé. Ce sont, par exemple, l'adénocarcinome du colon, l'adénocarcinome du rein, le carcinome corticosurrénalien, le phéochromocytome, les sarcomes de l'enfant et la leucémie secondaire. Cette liste n'est cependant pas exhaustive. Les expérimentations décrites ci-après illustreront la présente invention de manière plus détaillée, les exemples b) et d) n'étant mentionnés qu'à titre de comparaison.

### a) Mise en évidence in vitro de l'activité inhibitrice de la cinchonine

a.1. Des portions de 10'000 cellules provenant de la lignée DHD/K12/TRb ("drug resistant cell line"/MDR+) ont été réparties en séries et implantées sur lamelle, 48 heures avant traitement.

On a procédé à une première incubation de 60 minutes en milieu HAMF 10, répartissant les additifs comme suit .

| témoins | ---- |
|---------|------|
| série zéro | doxorubicine (DXR) à 10 $\mu$g/ml |
| série 1 | DXR (10 $\mu$g/ml) + cinchonine (5 $\mu$g/ml) |
| série 2 | DXR (10 $\mu$g/ml) + quinine (5 $\mu$g/ml) |
| série 3 | DXR (10 $\mu$g/ml) + vérapamil (5 $\mu$g/ml) |

Les séries 2 et 3 sont utilisées à titre comparatif, s'agissant de substances inhibitrices de la MDR connues. On a procédé ensuite à une seconde incubation, de 75 minutes, en milieu HAMF 10 additionné d'une nouvelle quantité de substance inhibitrice (séries 1 à 3). L'appréciation semi quantitative de la présence de DXR dans le cytoplasme des cellules traitées a été effectuée en microscopie de fluorescence (lecture double aveugle) pour donner les résultats ci-après.

| Témoins | 0 | série zéro | 0 |
| --- | --- | --- | --- |
| série 1 | +++ | série 2 | +++ |
| série 3 | +++ | | |
| 0 = absence de fluorescence | | | |
| + = unité arbitraire de fluorescence | | | |

On constate ainsi que la cinchonine exerce un effet inhibiteur de la MDR. Cet effet est comparable à celui constaté pour la quinine et le vérapamil.

a.2. On a procédé à une expérimentation comparable, à l'aide de portions de 10'000 cellules DHD/K12/TRb chacune, implantées 24 heures avant traitement.

Traitement proprement dit : incubation de 72 heures en milieu HAMF 10 + 10% SVF, en présence ou non de cinchonine. Pour ce qui est de l'agent cytotoxique, on a utilisé l'étoposide (VP 16), la vincristine, la vindésine, la mitoxantrone (MXN), respectivement la doxorubicine, à un taux constant. La concentration de cinchonine a par contre varié comme suit :

$$0 - 0,1 - 1 - 5 - 10 \; \mu mole$$

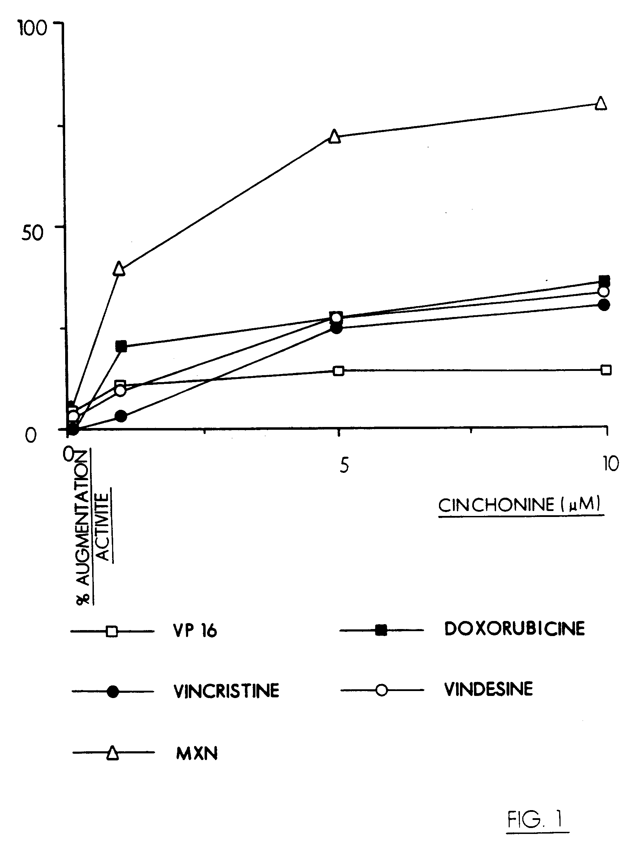

Les taux de survie des cellules implantées ont été appréciés par le moyen du test au bleu et convertis en % d'activité de la drogue antimitotique. Les résultats observés sont rassemblés dans Fig. 1. Ils confirment que la cinchonine exerce une activité inhibitrice de la MDR importante et potentialise l'activité cytotoxique de l'agent antimitotique utilisé.

a.3. On a procédé à une expérimentation analogue à la précédente, mais en vérifiant l'activité inhibitrice de la cinchonine sur d'autres lignées cellulaires tumorales coliques dont le produit de l'expression du gène mdr-1 et l'expression de la P-gp 180 ont été clairement établies.

Les cellules retenues ont été implantées 24 heures avant traitement, à raison de 10'000 cellules par puits. Le traitement proprement dit consiste en une incubation de 72 heures, en présence d'une dose de doxorubicine (DXR) de concentration constante, et d'une dose croissante de cinchonine, respectivement vérapamil :

$$0 - 1 - 5 - 10 - 20 - 40 \; \mu mole$$

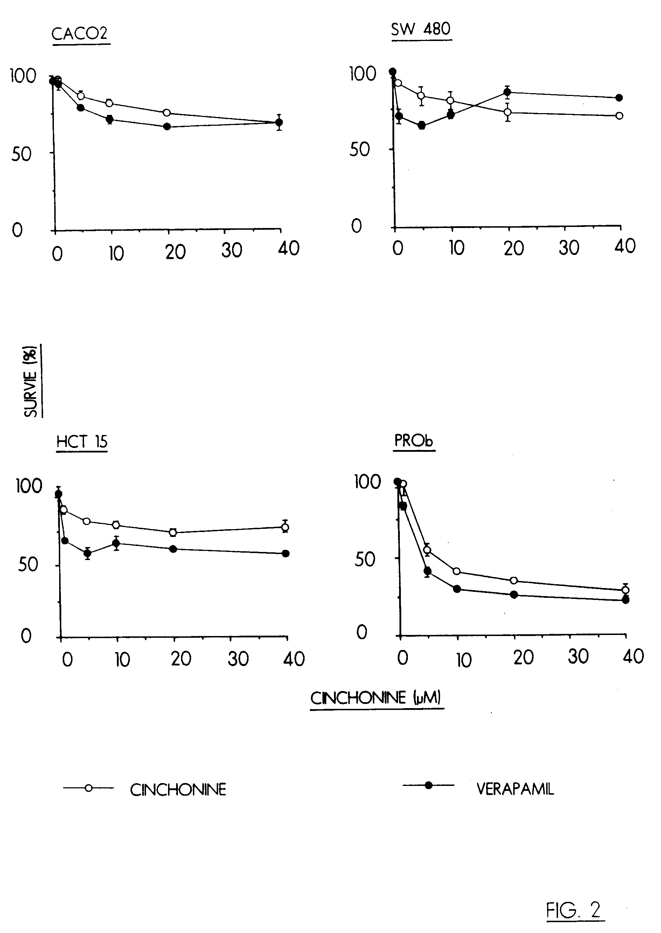

Les taux de survie cellulaire ont été déterminés au moyen du test au bleu et les résultats correspondants rassemblés dans la Fig. 2. Les lignées cellulaires utilisées furent les suivantes :

CACO2 - HCT15 - SW480 - PROb (DHD/K12/TRb)

Les résultats reportés dans Fig. 2 sont la moyenne de trois mesures. Ils confirment clairement l'activité inhibitrice de la cinchonine.

**b) Mise en évidence in vitro de l'activité inhibitrice de l'hydroquinidine**

Des portions de 100'000 cellules provenant de la lignée DHD/K12/TRb ("drug resistant cell line"/MDR+) ont été réparties en séries et implantées sur lamelle, 24 heures avant traitement comme indiqué ci-après, l'agent cytotoxique étant de la doxorubicine (DXR) contenant 3% de DXR 14C.

| témoins | ---- |
| --- | --- |
| série zéro | doxorubicine (DXR) à 10 $\mu$g/ml |
| série 1 | DXR (10 $\mu$g/ml) + hydroquinidine (15 $\mu$g/ml) |
| série 2 | DXR (10 $\mu$g/ml) + quinine (15 $\mu$g/ml) |
| série 3 | DXR (10 $\mu$g/ml) + vérapamil (15 $\mu$g/ml) |

Les séries 2 et 3 sont utilisées à titre comparatif, s'agissant de substances inhibitrices de la MDR connues. Après une incubation de deux heures, suivie de trois lavages successifs au moyen de PBS-BSA, trypsinisation et comptage de cellules, on a procédé à la mesure de la radioactivité intracellulaire à l'aide d'un compteur béta.

De ces mesures, on en a déduit un taux de doxorubicine intracytoplasmique directement lié à l'effet inhibiteur de la substance testée. Ces résultats sont rassemblés ci-après:

| Série no. | Agent | Radioactivité |
|---|---|---|
| 0 | DXR seule | 200 cpm |
| 1 | DXR + hydroquinidine | 875 cpm |
| 2 | DXR + quinine | 850 cpm |
| 3 | DXR + vérapamil | 1050 cpm |

On constate ainsi que l'hydroquinidine exerce un effet inhibiteur significatif de la MDR. Cet effet est pour le moins comparable à celui constaté pour la quinine.

**c) Mise en évidence ex vivo de l'activité inhibitrice de la cinchonine**

c.1. A divers groupes de 3 rats chacun, on a injecté par voie intra-veineuse l'une des deux substances inhibitrices ci-après, aux doses non toxiques maximales :

| cinchonine | 50 mg /kg (1) |
|---|---|
| quinine : | 40 mg /kg (2) |

Une heure après l'injection i.v., on a prélevé des échantillons sanguins de chaque sujet, par ponction cardiaque. On a procédé de même concernant des rats témoins, non traités préalablement.

c.2. Des portions de 10'000 cellules DHD/K12/TRb (voir a.1) ont été implantées 48 heures avant le traitement proprement dit comme indiqué ci-après, l'agent cytotoxique étant la doxorubicine (DXR).

| témoins | ---- |
|---|---|
| série zéro | DXR (10 $\mu$g/ml) + sérum rats témoins |
| série 1 | DXR (10 $\mu$g/ml) + sérum rats (1) |
| série 2 | DXR (10 $\mu$g/ml) + sérum rats (2) |

Après une première incubation d'une heure, on a procédé à 3 lavages successifs au moyen de HAMF 10, puis effectué une seconde incubation des cellules lavées durant une heure, en présence uniquement d'une nouvelle dose de sérum correspondant. L'appréciation semi quantitative de la rétention de DXR par les cellules traitées a été effectuée en microscopie de fluorescence (lecture double aveugle) pour donner les résultats ci-après.

| témoins | 0 | série zéro | 0 |
|---|---|---|---|
| série 1 | +++ | série 2 | ++ |

0 = absence de fluorescence
+ = unité arbitraire de fluorescence

On constate ainsi que la cinchonine est efficace ex vivo concernant l'effet inhibiteur de la MDR, pour le moins comparable à celui de la quinine.

**d) Mise en évidence ex vivo de l'activité inhibitrice de l'hydroquinidine**

d.1. A divers groupes de 3 rats chacun on a injecté par voie intra-péritonéale l'une des deux substances inhibitrices ci-après, aux doses suivantes :

| | |
|---|---|
| Hydroquinidine | 75 mg /kg (1) |
| Quinine | 75 mg /kg (2) |

Une heure après l'injection i.p., on a prélevé des échantillons sanguins de chaque sujet, par ponction cardiaque. On a procédé de même concernant des rats témoins, non traités préalablement.

d.2. Des portions de 100'000 cellules DHD/K12/TRb (voir a.1.) ont été implantées 24 heures avant le traitement proprement dit, comme indiqué ci-après, l'agent cytotoxique étant de la doxorubicine (DXR) contenant 3% de DXR 14C.

| | |
|---|---|
| témoins | ---- |
| série zéro | DXR (15 $\mu$g/ml) + sérum rats témoins |
| série 1 | DXR (15 $\mu$g/ml) + sérum rats (1) |
| série 2 | DXR (15 $\mu$g/ml) + sérum rats (2) |

Après une incubation de deux heures, suivie de trois lavages successifs au moyen de PBS-BSA, trypsinisation et comptage de cellules, on a procédé à la mesure de la radioactivité intracellulaire à l'aide d'un compteur béta.

De ces mesures, on en a déduit un taux de doxorubicine intracytoplasmique directement lié à l'effet inhibiteur de la substance testée. Ces résultats sont rassemblés ci-après:

| Série no. | Agent | Radioactivité |
|---|---|---|
| 0 | DXR seule | 250 cpm |
| 1 | DXR + hydroquinidine | 750 cpm |
| 2 | DXR + quinine | 500 cpm |

On constate ainsi, pour une même concentration sérique de substance inhibitrice, que le taux de DXR intracytoplasmique de la série 1 (DXR + hydroquinidine) est de trois fois supérieur à celui de la série zéro (DXR seule); de même, ce taux est environ une fois et demie supérieur à celui de la série 2 (DXR + quinine).

**e) Comparaison du pouvoir inhibiteur de la cinchonine par rapport à celui de la quinine par expérimentation ex vivo**

e.1. A divers groupes de 3 rats chacun on a injecté par voie intra-péritonéale l'une des trois substances inibitrices ci-après, aux doses suivantes :

| cinchonine | 75 mg /kg (1) |
|---|---|
| cinchonine | 100 mg /kg (2) |
| quinine | 75 mg /kg (3) |
| quinine | 100 mg /kg (4)* |

\* 2 rats morts sur 3

Une heure après l'injection i.p., on a prélevé des échantillons sanguins de chaque sujet, par ponction cardiaque. On a procédé de même concernant des rats témoins, non traités préalablement.

e.2. Des portions de 100'000 cellules DHD/K12/TRb (voir a.1.) ont été implantées 24 heures avant le traitement proprement dit en présence de l'agent cytotoxique (doxorubicine (DXR) contenant 3% de DXR 14C).

| témoins | ---- |
|---|---|
| série zéro | DXR (5 $\mu$g/ml) + sérum rats témoins |
| série 1 | DXR (5 $\mu$g/ml) + sérum rats (1) |
| série 2 | DXR (5 $\mu$g/ml) + sérum rats (2) |
| série 3 | DXR (5 $\mu$g/ml) + sérum rats (3) |
| série 4 | DXR (5 $\mu$g/ml) + sérum rats (4) |

Après une incubation de deux heures, suivie de trois lavages successifs au moyen de PBS-BSA, trypsinisation et comptage de cellules, on a procédé à la mesure de la radioactivité intracellulaire à l'aide d'un compteur béta.

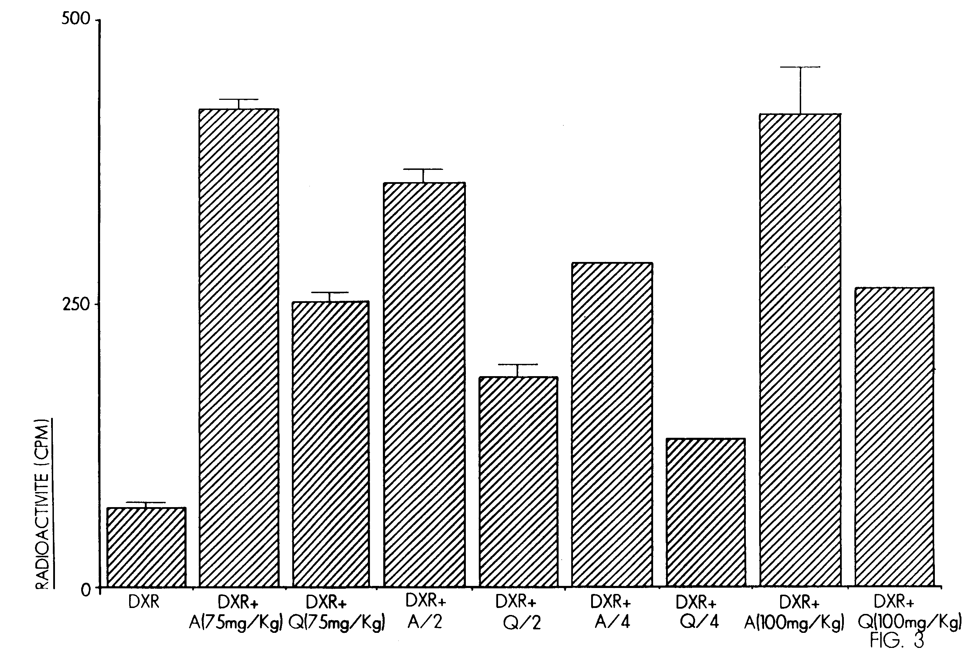

De ces mesures, on en a déduit un taux de doxorubicine intracytoplasmique directement lié à l'effet inhibiteur de la substance testée. Ces résultats sont rassemblés dans Fig. 3.

On constate ainsi, pour une même concentration sérique de substance inhibitrice, que le taux de DXR intracytoplasmique de la série 1 (DXR + cinchonine) est de six fois supérieur à celui de la série zéro (DXR seule); de même, ce taux est environ deux fois supérieur à celui de la série 3 (DXR + quinine), même après dilution des sérums d'un facteur 2, respectivement 4.

On observe en outre que la cinchonine est nettement moins toxique que la quinine : pour une injection i.p. de 100 mg/kg, deux sujets sur trois traités au moyen de quinine sont décédés après injection.

e.3. On a procédé à une expérimentation similaire, mettant en oeuvre une lignée cellulaire différente : K 562/ADM (myelogenous leukemia cell line).

Il se confirme également, dans ce cas-ci, que la cichonine témoigne d'une activité inhibitrice supérieure à celle de la quinine.

**f) Comparaison du pouvoir inhibiteur de la cinchonine par rapport à celui de la quinine par expérimentation in vivo.**

On a procédé à J0 à une injection intrapéritonéale de 1'000'000 de cellules DHD/K12/TRb par rat, afin d'induire chez les sujets la carcinomatose péritonéale d'origine colique utilisée comme modèle expérimental.

A J1, on a procédé à l'injection simultanée, par voie intrapéritonéale, de doxorubicine (DXR) libre et de substance inhibitrice, en solution dans du glucose aqueux à 5% et aux doses indiquées.

Groupe 1 :    DXR/quinine à la dose de 0,5 mg/kg DXR respectivement 80 mg/kg quinine.

Groupe 2 :    DXR/cinchonine à la dose de 0,5 mg/kg DXR, respectivement 80 mg/kg cinchonine

Groupe 3 :    DXR (0,5 mg/kg)

Groupe 4 :    témoins

Les groupes étaient constitués de 5 rats chacun. Ceux-ci ont été sacrifiés à J27 et, après autopsie, on a procédé à la mesure du poids des nodules tumoraux de chaque sujet. Les valeurs répertoriées ci-dessous sont des moyennes obtenues sur 5 sujet par groupe.

Groupe 1 : 0,2 g (± 0,2)

Groupe 2 : 0,1 g (± 0,1)

Groupe 3 : 4,8 g (± 1,2)

Groupe 4 : 5,0 g (± 1,2)

On constate ainsi que quinine et cinchonine inhibent significativement la résistance des tumeurs traitées au moyen de doxorubicine. Pour un dosage identique, la cinchonine se montre plus puissante que la quinine dans ce type d'expérimentation; l'avantage complémentaire réside dans la plus faible toxicité de la cinchonine comme démontré précédemment.

## Revendications

1. Utilisation de cinchonine dans la préparation de compositions pharmaceutiques destinées au traitement de tumeurs cancéreuses développant le phénomène de résistance multidrogue.

2. Utilisation selon la revendication 1, caractérisée en ce que la cinchonine se présente sous une forme stéréoisomérique pure, ou sous forme de mélanges de stétéoisomères.

3. Utilisation selon la revendication 1, caractérisée en ce que la cinchonine se présente sous une forme énantiomérique pure, ou sous forme de mélange d'énantiomères.

4. Utilisation selon l'une des revendications 1 à 3, caractérisée en ce que les compositions pharmaceutiques contiennent à titre d'agent inhibiteur de la résistance multidrogue de la cinchonine, ainsi que l'une au moins des substances choisies parmi l'amiodarone, la quinine, la quinidine, la cinchonidine, le vérapamil, la cyclosporine A, les céphalosporines, le bipéridène, la lidocaïne, la chlorpromazine, la pentazocine, la prométhazine, le potassium canrénoate, l'amitriptyline, le propanolol, le déméthoxyvérapamil, le diltiazème, la thioridazine, la trifluopéryzine, la chloroquine, la sbd-éthylène diamine, la réserpine, le tamoxifène, le torémifène, l'hydrocortisone, la progestérone, le salbutamol et leurs dérivés acylés ou esters.

5. Utilisation selon l'une des revendications 1 à 4, carctérisée en ce que l'agent inhibiteur de la résistance multidrogue est associé à un véhicule particulaire.

6. Utilisation selon la revendication 5, caractérisée en ce que le véhicule particulaire est une microcapsule, une nanocapsule, une microparticule ou une nanoparticule.

7. Utilisation selon la revendication 5, caractérisée en ce que le véhicule particulaire est un liposome.

## Claims

1. Use of cinchonine in the preparation of pharmaceutical compositions adapted to the treatment of cancer tumors developing the phenomenon of multiple drug resistance.

2. Use according to claim 1, characterized in that the cinchonine is present in a pure stereoisomic form, or in the form of mixtures of stereoisomers.

3. Use according to claim 1, characterized in that the cinchonine is present in a pure enantiomeric form, or in the form of mixtures of enantiomers.

4. Use according to one of claims 1 to 3, charcaterized in that the pharmaceutical compositions contain cinchonine as inhibitory agent against multiple drug resistance, and at least one substance selected from : amibdarone, quinine, quinidine, cinchonidine, verapamil, cyclosporin A, cephalosporins, biperiden, lidocaine, chlorpromazine, pentazocine, promethazine, potassium canrenoate, amitriptyline, propanolol, demethoxyverapamil, diltiazeme, thioridazine, trifluoperazine, chloroquine, sdb-ethylene diamine, reserpine, tamoxifen, toremifen, hydrocortisone, progesterone, salbutamol and their acylated derivatives or esters.

5. Use according to one of claims 1 to 4, characterized in that the agent for inhibiting multiple drug resistance is associated with a particulate vehicle.

6. Use according to claim 5, characterized in that the particulate vehicle is a microcapsule, a nanocapsule, a microparticle or a nanoparticle.

7. Use according to claim 5, characterized in that the particulate vehicle is a liposome.

**Patentansprüche**

1. Verwendung von Cinchonin in der Herstellung pharmazeutischer Zusammensetzungen für die Behandlung von Vielfachresistenz entwickelnden Krebsgeschwüren.

2. Verwendung gemäss Anspruch 1, dadurch gekennzeichnet, dass das Cinchonin in einer stereoisomer reinen Form oder in der Form von Stereoisomerengemischen vorliegt.

3. Verwendung gemäss Anspruch 1, dadurch gekennzeichnet, dass das Cinchonin in einer enantiomer reinen Form oder als Gemisch von Enantiomeren vorliegt.

4. Verwendung gemäss einem der Ansprüche 1 bis 3, dadurch gekennzeichnet, dass die pharmazeutischen Zusammensetzungen als Inhibitor der Vielfachresistenz Cinchonin sowie zumindest eine der Substanzen enthalten, die unter Amiodaron, Chinin, Chinidin, Cinchonidin, Verapamil, Cyclosporin A, den Cephalosporinen, Biperiden, Lidocain, Chlorpromazin, Pentazocin, Promethazin, Kaliumcanrenoat, Amitriptylin, Propranolol, Demethoxyverapamil, Diltiazem, Thioridazin, Trifluoperazin, Chloroquin, SDB-Ethylendiamin, Reserpin, Tamoxifen, Toremifen, Hydrocortison, Progesteron, Salbutamol und deren acylierten Abkömmlingen oder Estern ausgewählt wurde.

5. Verwendung gemäss einem der Ansprüche 1 bis 4, dadurch gekennzeichnet, dass der Inhibitor der Vielfachresistenz einem teilchenförmigen Träger verhaftet ist.

6. Verwendung gemäss Anspruch 5, dadurch gekennzeichnet, dass der teilchenförmige Träger eine Mikrokapsel, eine Nanokapsel, ein Mikropartikel oder ein Nanopartikel ist.

7. Verwendung gemäss Anspruch 5, dadurch gekennzeichnet, dass der teilchenförmige Träger ein Liposom ist.

FIG. 1

FIG. 2

FIG. 3

EP 0 526 608 B1